# EUROPEAN PATENT SPECIFICATION

(11) **EP 1 010 697 B1**
(45) Date of publication and mention of the grant of the patent: **31.10.2001**
(21) Application number: 99890261.3
(22) Date of filing: 05.08.1999
(51) Int. Cl.: C07D 305/14

(54) **Process for isolation of 14-b hydroxy-10-deacetyl baccatin-III**
Verfahren zu Isolierung von 14-b Hydroxy-10-desacetyl Baccatin III
Procédé pour l'isolation de 14-b hydroxy-10-désacétyl Baccatin III

(30) Priority: 15.12.1998 US 212321
(43) Date of publication of application: 21.06.2000
(73) Proprietor: Dabur Research Foundation, Sahibabad, Ghaziabad, 201 010 (IN)
(72) Inventor: Ramadoss, Sunder, Dr., Haus Khas, New Delhi 110016 (IN); Vardhan, Anand, Dr., Delhi 110 091 (IN)
(74) Representative: Schwarz, Albin, Dr.

(56) References cited:
- WO-A-94/07882
- US-A- 6 002 024

## Description

The present invention relates to the field of phyto-chemistry. More specifically, the invention provides a simple and cost-effective method for the isolation of the compound 14β-hydroxy-10-deacetyl baccatin-III (14β-OH-10-DAB) from the leaves of *Taxus baccata.* 14β-OH-DAB is an important intermediate compound useful in the preparation of 14β-OH paclitaxel and its analogues.

14β-hydroxy-10-deacetyl baccatin-III(14β-OH-DAB) is a very important intermediate useful in the synthesis of 14β-hydroxy-paclitaxel analogues, which show excellent cytotoxicity against human ovaries, lungs, colon and breast cancer cell hives [ref. J.Med. chem. 1977, 40, 267-278]. Several derivatives show better activity than those of paclitaxel and docetaxel.

14β-OH-10-deacetyl baccatin-III is represented by the following structural formula:

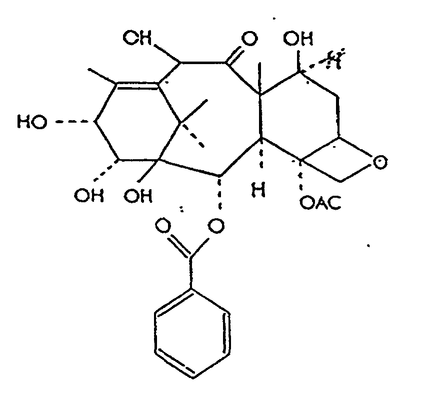

The compound 10-deacetyl baccatin-III is primarily found in the trunk and leaves of the plants of *Taxus sp.* in highest concentration. While the other parts of the plant also contain this compound, the concentration is less. Further, it is easier to extract 14β-Hydroxy-10-DAB from other plant parts than the leaves.

Most common methods for the isolation of 14β-hydroxy-10-DAB involve the extraction of biomass with alcohol or its mixture with less polar solvent to yield an extract which is semipurified by solvent maceration and finally chromatography to yield 14β-Hydroxy-10-DAB. Some of the common methods employed in the art for extraction of 14β-Hydroxy-10-DAB are given hereinbelow.

### Prior art references

WO-A-94/07882 discloses a method of obtaining 10-deacetyl saccatine III from different parts of the yew tree (Taxus sp.) by extraction and selective crystallization from methanol extract of the plant mass. Although the as the first method of isolation of 10-deacetyl -baccatin III without the use of any chromatographic separation, it does not proceed to the isolation of the 14-6-04-deacetyl-baccatin III.

European Patent No.0 559 019 A1 discloses a process for the isolation of 14β-hydroxy-10-deacetyl-baccatin III. However, the above process involves a tedious step of chromatography which adds to the cost of the process, especially when employed on a commercial scale.

US Patent No.5,453,520 discloses another method for the isolation of 14β-hydroxy-10-deacetylbaccatin III and its derivatives as antitumor agents. Again, this Patent discloses alcoholic extraction from *Taxus Wallichiana* leaves and isolation of 14β-hydroxy-10-deacetylbaccatin III using chromatography techniques.

Patent No. WO 9629321 A1 discloses 10-deacetylbaccatin III and 14β-hydroxy-10-deacetyl baccatin III derivatives and pharmaceutical compositions containing them. This patent is directed to synthesis of analogues of 14β-hydroxy-10-DAB & 10-DAB.

Another process for the extraction of 14β-hydroxy-10-DAB is published in Journal of Chem. Soc. Perkin Trans. 1, 1992 pg 2925. The process teaches solvent extraction of 14β-hydroxy-10-DAB from the leaves of *T*.*wallichiana* using methanol followed by silica gel chromatography and subsequent crystallization to yield 14β-hydorxy-10-DAB as a white powder.

In general, the above mentioned known methods employed for the isolation of 14β-hydroxy-10-DAB require the long, tedious and costly chromatography techniques. In these processes, the semipurified extracts of taxane species are subjected to tedious technique of absorbent chromatography which results in poor yield of 10-DAB and 14β-hydroxy-10-DAB.

Hence, there is a need to devise a process, which obviates the drawbacks of the conventional methods and also provides better yield of 14β-Hydroxy-10-DAB.

### Objects of the present Invention :

Accordingly, one objective of the present invention is to provide a simple and cost-effective process for the isolation 14β-OH-10-DAB (14β-OH-10deacetylbaccatin-III) for useful as an intermediate in the synthesis of 14β-hydroxy paclitaxel analogues.

Another objective of the present invention is to provide a process which does not involve tedious step of isolation by chromatography techniques at any stage for isolation of 14β-OH-10-DAB.

Yet another objective of the present invention is to provide a process wherein the solvent used in various steps can be recycled.

It is also an objective of the present invention to provide a novel process which can be used for extraction of 14β-OH-10-DAB from any part of the plant of *Taxus* species.

### Summary of the invention:

In accordance with the above and other objects, the present invention provides a novel process for the isolation of 14β-OH-10-DAB from the plant parts of Taxus species, comprising the steps of :
1) Preparing an alcoholic extract of the dried leaves of *Taxus baccata;* treatment thereof with aliphatic ketones; evaporation of the mother liquor and selective crystallization for isolation of 10-DAB, and
2) Further treatment of mother liquor obtained after 10-DAB crystallization with nitrile solvents followed by aromatic hydrocarbon solvents and then selective crystallization and isolation of 14β-OH-10-DAB.

### Detailed description of the Invention:

The present invention provides a novel process for the isolation of 14β-hydroxy-10-deacetyl-baccatin III, a compound used for the synthesis of 14β-hydroxy paclitaxel analogues.

The process is simple and 14β-OH-10-deacetyl baccatin III can be selectively isolated from the recoverable part of a plant of *Taxus species* by crystallization. The present process involves the steps of:
(a) extracting the pulverised and optionally dried parts of a plant of *Taxus species,* with aliphatic alcohol,
(b) preparing a partially concentrated alcoholic extract containing 10-DAB, and minor quantity of 14β-OH-10-DAB,
(c) treating extract with aliphatic ketones and separating the insolubles from the mother liquor by centrifugation or filtration,
(d) treating the residue obtained after evaporation of the mother liquor of step (c) under vacuum with a mixture of aliphatic ketones and water, and removing the insolubles through a celite bed by filtration or centrifugation,
(e) extraction of the aqueous ketonic solution with aromatic hydrocarbons to remove less polar substances, followed by extraction with water immiscible aliphatic esters or chlorinated solvents,
(f) evaporating the organic layer of (e) to dryness to obtain a semisolid residue from which crude 10-DAB is obtained by selective crystallizations,
(g) isolation and purification of 10-DAB,
(h) evaporation of mother liquor of (g) and followed by treatment of the residue with nitrile and aromatic hydrocarbon solvent, and
(i) isolation and purification of 14β-OH-10-DAB by solvent crystallization from the residue obtained after treatment with nitrile solvent followed by aromatic hydrocarbon solvents.

In one embodiment, the aliphatic alcohol used for preparation of alcoholic extract is selected from the group consisting of methanol, ethanol, propanol, isopropanol and tertiary butanol.

In one feature, the aliphatic alcohol is methanol.

In another embodiment, the alcoholic extract is obtained by stirring the pulverised and optionally dried parts of the plant of *Taxus species* in methanol, ethanol, propanol, isopropanol or butanol.

In another feature of the invention, the crystallization of 10-DAB is carried out by treatment of the residue with aliphatic nitrile solvents optionally mixed with aliphatic alcohols selected from the group consisting of methanol, isopropanol, butanol, or aliphatic esters selected ethyl acetate, propionyl acetate and isoamyl acetate. The aliphatic nitriles may be selected from acetonitrile and propionitrile.

In yet another feature, 10-DAB and 14β-OH-10-DAB is extracted from any part of any plant species of the genus *Taxus.*

In a feature, 14-β-OH-10-DAB is isolated by filtration and crystallization.

In another feature, 10-DAB is isolated by crystallization followed by centrifugation.

In yet another feature, the plant is *Taxus baccata.*

In another feature, the parts of the plant are the leaves.

The process illustrated above for isolation of 14β-hydroxy-10-deacetyl-baccatin III does not involve any chromatographic technique at any stage.

The preferred process comprises of the following steps:
1. The leaves of *Taxus baccata* are pulverised and may be optionally dried. Water miscible aliphatic alcohol selected from methanol, ethanol, propanol, isopropanol, butanol, is added to ground leaves and the mixture is stirred for 12 hours. The preferred aliphatic alcohol used in the present process is methanol.
2. The semi-concentrated alcoholic extract so obtained contains 10-DAB, 14β-OH-10-DAB and other taxanes. The said extract is stirred with aliphatic ketone for one hour. The insoluble material is then separated out by filtration through a celite bed or centrifugation. The liquid left behind is the mother liquor which is then used in further processing.
3. Mother liquor of step (2) is evaporated under vacuum to yield a residue which is treated with a mixture of aqueous ketonic solvents and the insolubles are removed by filtration or centrifugation through a celite bed. Clear aqueous ketonic solution may be used for further processing.
4. Clear aqueous ketonic solution obtained is extracted with aromatic hydrocarbon to remove less polar substances other than 10-DAB. Then the remaining aqueous ketonic phase is treated with water immiscible solvent, preferably, aliphatic ester or chlorinated solvent, to extract 10-DAB, along with 14β-hydroxy-10-DAB.
5. Thereafter, the organic solvent extract is evaporated under vacuum to dryness at 80°-90°C to yield a semisolid residue. Subsequently, 10-DAB is selectively crystallised from the residue obtained above by treating it with aliphatic nitrile solvent such as acetonitrile, propionitrile optionally mixed with an aliphatic alcohol such as methanol, isopropanol, n-butanol or aliphatic ester such as acetone, ethyl acetate, butyl acetate. It is advantageous to perform the selective crystallization in acetonitrile, optionally in the presence of ethanol, methanol or ethyl acetate, and /or butyl acetate. The 10-DAB is purified by any conventional method such as crystallization followed by centrifugation.
6. After the crystallization and isolation of 10-DAB, the mother liquor is slightly enriched in 14β-OH-10-DAB. The liquor is evaporated to dryness and treated with nitrile solvent followed by aromatic hydrocarbon at 80°C for 1-2 hours and cooled to room temperature. The insolubles are removed and its treatment with aliphatic alcohol or aliphatic ester or chlorinated solvent to yield crude 14β-OH-10-DAB (preferably alcoholic solvent). The pure 14β-OH-10-DAB is obtained by solvent crystallization employing aliphatic alcoholic or ester solvents.

The above novel process for the isolation of 14β-OH-10-DAB can be applied for extraction from any part of the plant *Taxus*.*sp*. esp *T. baccata, T*.*brevifolia*, *T.cannadensis, T. cuspidata, T.floridara, T. media or T. wallichiana.* The process described above, is simple and does not involve any chromatography at any stage and 14β-OH-10-DAB can be obtained using only solvents.

The other advantage of the present process is that the solvents used in various steps can be reused. In addition, the applicant has identified that though the raw material used in the present invention contains 10-DAB in higher quantity than 14-β-OH-10-DAB, the isolation of the latter could be achieved without implementing chromatographic procedures.

The above novel process is described in detail by the following examples which are provided for illustration only and should not be construed to limit the scope of the present invention.

### Example -I

The leaves of *Taxus baccata* are pulverised and optionally dried. Preferably, the mean particle size of the leaves is close to 0.6 mm to 0.8 mm. An alcoholic extract is prepared by stirring 1000 L of methanol with 100 kg. of the ground the leaves (rotation of 58 per minute) at ambient temperature in the reactor for 12 hrs. The methanolic solution collected after centrifugation is evaporated under reduced pressure (150 m bar) at 40-50°C in an evaporating reactor to obtain a semi concentrated extract whose weight is between 20-30% of the weight of leaves.

The 25 kg. of semiconcentrated methanolic extract containing 0.12% of 10-deacetyl baccatin-III, 20 % of water and 10% of methanol (prepared under the conditions described above) is stirred (58 rpm) with 175 L. (liter) of acetone at ambient temperature for 1 to 2 hrs. The insoluble solid that appears is separated by filtration or centrifugation. The acetone soluble in mother liquor forming 35 % of the weight of the semi concentrated extracted is distilled off to dryness at 40-50°C under vacuum (150 m bar).

8.75 kg of dry residue obtained is stirred with the 43.75 L. of acetone and demineralized water (DM) mixture (ratio preferably 2:8) for 2 hr. at ambient temperature at high rpm (72 revolution per minute). The insoluble material is removed by filtration or centrifugation on celite bed. The aqueous ketonic mother liquor collected is clear dark red.

The aqueous ketonic layer is extracted twice with 17.5 L. and once with 8.75 L. (liter) of toluene by stirring (40 revolution per minute) for 1 hour each extraction.

The aqueous ketonic layer obtained after toluene extraction, is then extracted with methylene chloride (4 X 16.5L) at ambient temperature for 30 minute each extraction by stirring at(40 revolution per minute). The methylene chloride, layer is combined and dried over sodium sulphate and then evaporated to dryness under vacuum (150 m bar) at 25° to 65°C in a evaporating flask. The weight of the methylene chloride residue usually lies between 1.5 to 1.8 w/w% of the semi concentrated methanol extract, containing 4.5 to 6% of 10- deacetyl baccatin III.

### Example II

468.7 g of dry extract containing 5.1% of 10-deacetyl baccatin-III is obtained under the conditions described in example I. The extract is stirred 40 revolution for minute with 975 ml of acetonitrile at 50-60°C. When the extract is completely dissolved, the mixture is cooled at 0-5°C for 12-16 hrs. and stirred at very low rpm (20). The solid insoluble are separated out by filtration or centrifugation and washed with 50 ml of acetonitrile. A crude solid (29.43g) is obtained after drying it at reduced pressure (0.5 m bar) at 60-70°C for 12 hrs. having 76.1% of 10-deacetyl baccatin-III.

### Example III

29.43g of crude solid obtained under the condition described in example II, are dissolved in 735.5 ml of methanol by refluxing at 70°C for 1 hr with stirring (rpm-58). The solution is cooled to ambient temperature and then filtered through a celite bed to obtain clear solution. The filtrate is stirred at very low rpm (20) and 295 ml of acetonitrile is slowly added to it. The mixture is then cooled to 0-5°C and maintained for 12-15 hrs. The precipitate is separated by filtration or centrifugation and washed with 10 ml mixture of methanol-acetonitrile (1:1). The product is dried at reduced pressure (0.5m bar) at 90-95°C for 20-30 hr. 22.2 g of off white final product is thus obtained, containing 94.1% of 10-deacetyl baccatin-III.

The mother liquor obtained after final crystallization is evaporated to dryness, and recycled for purification of 10-deacetyl baccatin-III.

### Example IV

30 g of crude solid obtained under the condition described in Example II, is dissolved in 750 ml of acetone by refluxing at 65°C for 1 to 2 hr. The solution is cooled to room temperature and then filtered through a celite bed. Filtrate is stirred at very low rpm (20) and acetonitrile (200 ml) is slowly added to it. Temperature of the solution is brought down to 0-5°C and maintained up to 15-20 hrs. The precipitate is separated out by centrifuging and washing the solid with 1:1 mixture of acetone and acetonitrile. The final product is dried under vacuum (0.5 m bar) at temperature 80-90°C for 12-20 hrs. The final (23.1 g) product is thus obtained, containing 93.5% of 10-deacetyl baccatin-III.

### Example V

After the purification of 10-deacetyl baccatin-III, the mother liquor obtained above is evaporated under vacuum (75 m bar) at 70-80°C to dryness. The residue (35g) obtained is then stirred with acetonitrile (350 ml) at 70-80°C for 1 hour. The solution is chilled for 10 hours at 0-5°C and then insoluble material is pump filtered. The mother liquor obtained is evaporated to dryness under reduced pressure (150 m bar) at 70-80°C. The residue (25g) obtained is then stirred to 58 rpm at 80°C with 75 ml of toluene for 1 hour. The temperature of mixture is brought down to room temperature, separated the toulene soluble portion from toulene insoluble residue which is heated at 80°C under vacuum to remove residual solvent. Toulene insoluble resiude is then dissolved in one equivalent of methanol by stirring at 70°C. The solution is kept for chilling at 0-5°C for 24-48 hours. Crude solid is filtered and vacuum dried at 70-90°C for 12 hours to obtain 1.9 g of solid 14β-OH-10-DAB.

### Example VI

The crude solid 14β-OH-10-deacetyl baccatin-III (prepared under the conditions described in example V) 1.9 g is dissolved in 47.5 ml of ethylacetate by refluxing for 1-2 hours. The solution is cooled to room temperature (RT) and then filtered through a celite bed. The clear filtrate obtained is distilled off the solvent under vacuum (150m bar) at 40-60°C to reduce the volume up to 24 ml and then kept at 0-5°C up to 12-24 hours. The crystallised solid is pump filtered, washed with chilled ethylacetate (2ml) and vacuum dried at 90°C for 12 hours, obtained 1.28 g of furnished 14-β-OH-10-deacetyl baccatin-III.

### Example VII

The crude solid of 14β-OH-10-deacetyl baccatin-III 1.9 g (prepared under the same condition described in example V) is dissolved in 41.8 ml of methanol by refluxing for 1 hour with stirring. The solution is cooled to room temperature (RT) and then filtered through a celite bed. Clear filtrate is kept for chilling 0-5°C for 12 hours. Crystallised solid is filtered, vacuum dried at 75-90°C for 12 hours and thereby obtaining 1.12 g of finished 14β-OH-10-deacetyl baccatin-III

### Advantages of the novel process :

a) It is simple, cost effective and has commercial feasibility.
b) It does not involve tedious process of chromatographic technique at any stage of this process.
c) In this process, there is reusability of the solvent in many steps.
d) This process is applicable for extraction of any part of the plant, of different species of *Taxus.*
e) This process can be used to separate placitaxel present in the primary alcoholic extract.
f) Depending on the quality of raw material yield of 10-deacetyl baccatin-III varies from 60 % to 90 % of its content in the raw material.
g) Though 14β-OH-DAB is present in minor quantity at the methanolic extract level, it is possible to isolate this compound without using any chromatographic technique at any stage.

## Claims

1. A process for the isolation of 14β-OH-10-deacetyl baccatin III from the recoverable part of a plant of Taxus species by crystallization, comprising the steps of:
(a) extracting the pulverised and optionally dried parts of a plant of *Taxus species,* with aliphatic alcohol,
(b) preparing a partially concentrated alcoholic extract containing 10-DAB, and minor quantity of 14β-OH-10-DAB,
(c) treating extract with aliphatic ketones and separating the insolubles from the mother liquor by centrifugation or filtration,
(d) treating the residue obtain after evaporation of the mother liquor of step (c) under vacuum with a mixture of water and aliphatic ketonic solvent and removing the insolubles through a celite bed by filtration or centrifugation,
(e) extraction of the aqueous ketonic solution with aromatic hydrocarbons to remove less polar substances, followed by extraction with water immiscible aliphatic esters or chlorinated solvents,
(f) evaporating the organic layer of (e) to dryness to obtain a semisolid residue from which crude 10-DAB is obtained by selective crystallizations,
(g) isolation and purification of 10-DAB,
(h) evaporation of mother liquor of (g), followed by treatment of the residue with nitrile and aromatic hydrocarbon solvent, and
(i) isolation and purification of 14β-OH-10-DAB from the residue obtained after treatment with nitrite solvents followed by aromatic hydrocarbon solvents.

2. A process as claimed in claim 1, wherein the aliphatic alcohol used for preparation of alcoholic extract is selected from the group consisting of methanol, ethanol, propanol, isopropanol and butanol.

3. A process as claimed in claim 1, wherein the aliphatic alcohol used is methanol.

4. A process as claimed in claim 1, wherein the alcoholic extract is obtained by stirring the pulverised and optionally dried parts of the plant of the Taxus species in methanol, ethanol, propanol, isopropanol or butanol.

5. A process as claimed in claim 1, wherein the selective crystallization of 10-DAB is carried out by treating of the semisolid residue with aliphatic nitrile solvent optionally mixed with aliphatic alcohols.

6. A process as claimed in claim 1, wherein the aliphatic nitrile solvent is selected from acetonitrile or propionitrile.

7. A process as claimed in claim 1, wherein the nitrile solvent is mixed with aliphatic alcohols selected from the group consisting of methanol, isoporpanol, butanol or with aliphatic esters selected from ethyl acetate, propionyl acetate and isoamyl acetate.

8. A process as claimed in claim 1, wherein 10-DAB and 14β-OH-10-DAB is extracted from any part of any plant of the genus *Taxus.*

9. A process as claimed in claim 1, wherein 14-β-OH-10-DAB is isolated by filtration and crystallization.

10. A process as claimed in claim 1, wherein 10-DAB is isolated by crystallization followed by centrifugation.

11. A process as claimed in claim 1, wherein the plant is *Taxus baccata.*

12. A process as claimed in claim 1, wherein the parts of the plant are the leaves.

## Patentansprüche

1. Verfahren zur Isolierung von 14β-OH-10-Desacetyl baccatin-III aus dem regenerierbaren Teil einer Pflanze der Taxus-Spezies durch Kristallisation, umfassend die nachstehenden Schritte:
(a) Extrahieren der pulverisierten und gegebenenfalls getrockneten Teile einer Pflanze der *Taxus-Spezies* mit aliphatischem Alkohol,
(b) Herstellen eines teilweise konzentrierten alkoholischen Extrakts, der 10-DAB und eine geringe Menge an 14β-OH-10-DAB enthält,
(c) Behandeln des Extrakts mit aliphatischen Ketonen und Trennen der unlöslichen Substanzen von der Mutterlauge durch Zentrifugation oder Filtration,
(d) Behandeln des nach einer Evaporation der Mutterlauge nach Schritt (c) unter Vakuum erhaltenen Rests mit einer Mischung aus Wasser und einem aliphatischen ketonischen Lösungsmittel und Entfernen der unlöslichen Substanzen durch ein Kieselgur-Bett durch Filtration oder Zentrifugation,
(e) Extrahieren der wässerigen ketonischen Lösung mit aromatischen Kohlenwasserstoffen, um weniger polare Substanzen zu entfernen, gefolgt von einer Extraktion mit mit Wasser nicht mischbaren aliphatischen Estern oder chlorierten Lösungsmitteln,
(f) Evaporieren der organischen Schicht nach (e) bis zur Trockenheit, um einen halbfesten Rest zu erhalten, aus dem Roh-10-DAB durch selektive Kristallisationen erhalten wird,
(g) Isolierung und Reinigung von 10-DAB,
(h) Evaporation der Mutterlauge nach (g), gefolgt von einer Behandlung des Rests mit einem Nitril-Lösungsmittel und einem Lösungsmittel eines aromatischen Kohlenwasserstoffs, und
(i) Isolierung und Reinigung von 14β-OH-10-DAB aus dem Rest, der nach der Behandlung mit Nitril-Lösungmitteln, gefolgt von Lösungmitteln aromatischer Kohlenwasserstoffe, erhalten wurde.

2. Verfahren wie in Anspruch 1 beansprucht, wobei der zur Herstellung des alkoholischen Extrakts verwendete aliphatische Alkohol aus der Gruppe bestehend aus Methanol, Ethanol, Propanol, Isopropanol und Butanol ausgewählt ist.

3. Verfahren wie in Anspruch 1 beansprucht, wobei der verwendete aliphatische Alkohol Methanol ist.

4. Verfahren wie in Anspruch 1 beansprucht, wobei der alkoholische Extrakt durch Rühren der pulverisierten und gegebenenfalls getrockneten Teile der Pflanze der Taxus-Spezies in Methanol, Ethanol, Propanol, Isopropanol oder Butanol erhalten wird.

5. Verfahren wie in Anspruch 1 beansprucht, wobei die selektive Kristallisation von 10-DAB durch ein Behandeln des halbfesten Rests mit einem aliphatischen Nitril-Lösungsmittel, das gegebenenfalls mit aliphatischen Alkoholen vermischt ist, durchgeführt wird.

6. Verfahren wie in Anspruch 1 beansprucht, wobei das aliphatische Nitril-Lösungsmittel aus Acetonitril oder Propionitril ausgewählt ist.

7. Verfahren wie in Anspruch 1 beansprucht, wobei das Nitril-Lösungsmittel mit aliphatischen Alkoholen vermischt ist, die ausgewählt sind aus der Gruppe bestehend aus Methanol, Isopropanol, Butanol, oder mit aliphatischen Estern, die ausgewählt sind aus Ethylacetat, Propionylacetat und Isoamylacetat.

8. Verfahren wie in Anspruch 1 beansprucht, wobei 10-DAB und 14β-OH-10-DAB aus einem beliebigen Teil einer beliebigen Pflanze der Gattung *Taxus* extrahiert werden.

9. Verfahren wie in Anspruch 1 beansprucht, wobei 14β-OH-10-DAB durch Filtration und Kristallisation isoliert wird.

10. Verfahren wie in Anspruch 1 beansprucht, wobei 10-DAB durch eine Kristallisation, gefolgt von einer Zentrifugation, isoliert wird.

11. Verfahren wie in Anspruch 1 beansprucht, wobei die Pflanze *Taxus baccata* ist.

12. Verfahren wie in Anspruch 1 beansprucht, wobei die Teile der Pflanze die Blätter sind.

## Revendications

1. Procédé pour l'isolement de la 14β-OH-10-désacétylbaccatine III à partir de la partie récupérable d'une plante de l'espèce *Taxus* par cristallisation, comprenant les étapes consistant à :
(a) extraire les parties pulvérisées et éventuellement séchées d'une plante de l'espèce *Taxus* avec un alcool aliphatique,
(b) préparer un extrait alcoolique partiellement concentré contenant la 10-DAB, et une quantité minoritaire de 14β-OH-10-DAB,
(c) traiter l'extrait par des cétones aliphatiques et séparer les matières insolubles de la liqueur mère par centrifugation ou filtration,
(d) traiter le résidu obtenu après évaporation de la liqueur mère de l'étape (c) sous vide avec un mélange d'eau et d'un solvant cétonique aliphatique et éliminer les matières insolubles au travers d'un lit de Célite par filtration ou centrifugation,
(e) extraire la solution aqueuse cétonique par des hydrocarbures aromatiques pour éliminer les substances moins polaires, puis extraire par des esters aliphatiques ou des solvants chlorés non miscibles à l'eau,
(f) évaporer la couche organique de (e) à sec pour obtenir un résidu semi-solide à partir duquel la 10-DAB brute est obtenue par cristallisations sélectives,
(g) isoler et purifier la 10-DAB,
(h) évaporer la liqueur mère de (g), puis traiter le résidu par un nitrile et un solvant hydrocarboné aromatique, et
(i) isoler et purifier la 14β-OH-10-DAB à partir du résidu obtenu après le traitement par des solvants nitriles puis par des solvants hydrocarbonés aromatiques.

2. Procédé selon la revendication 1, dans lequel l'alcool aliphatique utilisé dans la préparation de l'extrait alcoolique est choisi dans le groupe constitué par le méthanol, l'éthanol, le propanol, l'isopropanol et le butanol.

3. Procédé selon la revendication 1, dans lequel l'alcool aliphatique utilisé est le méthanol.

4. Procédé selon la revendication 1, dans lequel l'extrait alcoolique est obtenu par agitation des parties pulvérisées et éventuellement séchées de la plante de l'espèce *Taxus* dans le méthanol, l'éthanol, le propanol, l'isopropanol ou le butanol.

5. Procédé selon la revendication 1, dans lequel la cristallisation sélective de la 10-DAB est réalisée par traitement du résidu semi-solide par un solvant nitrile aliphatique éventuellement mélangé à des alcools aliphatiques.

6. Procédé selon la revendication 1, dans lequel le solvant nitrile aliphatique est choisi parmi l'acétonitrile ou le propionitrile.

7. Procédé selon la revendication 1, dans lequel le solvant nitrile est mélangé à des alcools aliphatiques choisis dans le groupe constitué par le méthanol, l'isopropanol, le butanol ou à des esters aliphatiques choisis parmi l'acétate d'éthyle, l'acétate de propionyle et l'acétate d'isoamyle.

8. Procédé selon la revendication 1, dans lequel la 10-DAB et la 14β-OH-10-DAB sont extraites d'une partie quelconque d'une plante quelconque du genre *Taxus.*

9. Procédé selon la revendication 1, dans lequel la 14β-OH-10-DAB est isolée par filtration et cristallisation.

10. Procédé selon la revendication 1, dans lequel la 10-DAB est isolée par cristallisation suivie d'une centrifugation.

11. Procédé selon la revendication 1, dans lequel la plante est *Taxus baccata*.

12. Procédé selon la revendication 1, dans lequel les parties de la plante sont les feuilles.
